# EUROPEAN PATENT APPLICATION

(11) **EP 1 779 775 A1**
(43) Date of publication of application: **02.05.2007**
(21) Application number: 05766386.6
(22) Date of filing: 15.07.2005
(51) Int. Cl.: A61B 5/0478

(54) **BRAIN FUNCTION DETECTOR**

(30) Priority: 03.08.2004 JP 2004226442
(71) Applicant: Olympus Corporation, Tokyo 151-0072 (JP); Brain Functions Laboratory, Inc., Kawasaki-shi, Kanagawa (JP)
(72) Inventor: KUMADA, Yoshiyuki, 1670053 (JP); MUSHA, Toshimitsu, C/O Brain Functions Lab., Inc., Kawasaki-shi, Kanagawa (JP)
(74) Representative: von Hellfeld, Axel
(86) International application number: PCT/JP2005/013165
(87) International publication number: WO 2006/013716

(57) **Abstract**

It is possible for the tips of all the electrodes to make contact with the scalp with greater reliability even with respect to different subjects, by adaptation to individual differences in the heads of subjects, and the functional status of the brain can be detected with greater accuracy. A brain function detection apparatus is provided which has a hat-shaped apparatus main body for mounting on the head of a subject, a plurality of electrodes for detecting brain wave signals, which are mounted in the apparatus main body separated from each other at intervals, and which are arranged in contact with the scalp, and the apparatus main body has a plurality of holder pieces in which at least one electrode is mounted, a bendable belt for linking the holder pieces, and a holder piece interval-adjusting apparatus for adjusting the distance between the holder pieces.

## Description

### [Technical Field]

The present invention relates to an apparatus for detecting brain function.

### [Background Art]

Conventionally, with the goal of the early detection of Alzheimer's disease, analysis of the functional status of the brain has been performed and various analytical methods of the functional status of the brain have been proposed (for example, refer to Non-patent Document 1 and Non-patent Document 2). In these analytical methods, brain wave signals are detected and judgments are made based on the smoothness of the spatial transmission of the brain wave signals and the standard deviation thereof. When the activity of the brain nerve cells is normal, the brain wave signals are transmitted smoothly and the variation therein is small, but when Alzheimer's disease is progressing and the activity of brain nerve cells has deteriorated, there is a tendency for the smoothness of the spatial transmission of brain wave signals to be lost and for the standard deviation thereof to increase.

In order to carry out this type of analysis with good accuracy, it is essential for the brain wave signals to be detected with good accuracy. Brain wave signals are detected by bringing a plurality of electrodes into contact with the scalp, but due to the presence of hair and lack of uniformity in the shape of the head, it is common for the electrodes to be brought into contact with the scalp by means of a paste in order to realize a reliable state of contact between the electrodes and the scalp. However, when past is used, there are the problems that a considerable amount of time is necessary prior to initiation of the detection, placing a large burden on the subject being examined, and in addition, that after the detection, when the electrodes are removed, procedures such as washing of the hair are necessary for removing the paste, placing a large burden on the subject being examined.

However, paste-less detection methods for brain wave signals, in which a paste is not used, have also been proposed (for example, refer to Patent Document 1 and Patent Document 2). These detection methods are methods in which a fibrous piece into which an electroconductive solution has been absorbed or a foam material impregnated with a hydrated gel which provides conductivity are brought into contact with the scalp as electrodes, respectively. Since a paste is not used, there is the benefit that the time required for mounting and removal is reduced.

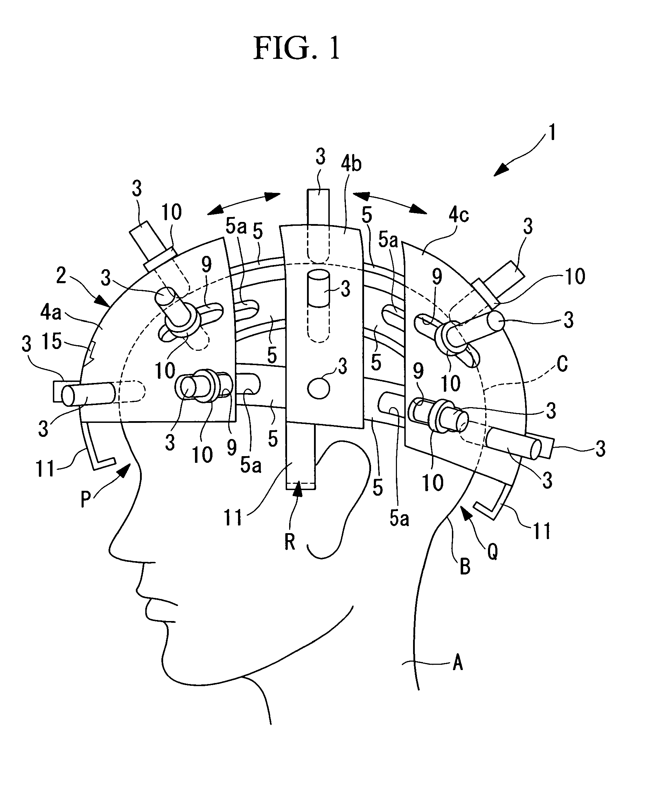

[Non-Patent Document 1] Brain Functions Laboratory, Inc., "DIMENSION New brain wave analysis method for detecting neuronal dysfunction early in Alzheimer's disease", online, Search Date: March 8, 2004; Internet URL: http://www.bfl.co.jp/abst-32.html
[Non-Patent Document 2] Brain Functions Laboratory, Inc., "DIMENSION Diagnosis method of neuronal dysfunction, Online, Search Date: March 8, 2004; Internet URL: http://www.bfl.co.jp/abst-3l.html
[Patent Document 1] Japanese Unexamined Patent Application, First Publication No. Hei 10-165386 (page 2, and others)
[Patent Document 2] Japanese Unexamined Utility Model Application, First Publication No. Hei 2-63811 (FIG. 1, and others)

### [Disclosure of Invention]

It is necessary for the detection of brain waves to be carried out by correctly arranging a plurality of electrodes, and a method in which the electrodes are fixed in position by means of a hat-shaped holder which is put onto the head of the subject being examined can be considered. However, when the hat-shaped holder is a helmet-shaped rigid holder, since the size and shape of the head of the subject being examined differs for each subject being examined, it is difficult to arrange the electrodes at correct positions when fitting the same holder onto many subjects. On the other hand, when the hat-shaped holder is a flexible holder, while it is possible to fit the holder on a plurality of subjects, it is difficult to maintain the electrodes in position in a predetermined positional relationship.

The present invention was achieved in consideration of the above-mentioned circumstances, and the present invention has objects of providing an apparatus for detecting brain function which is adaptable to individual differences in the heads of subjects, which makes it possible for the tips of all the electrodes to make contact with the scalp with greater reliability even with respect to different subjects, and with which the functional status of the brain is detectable with greater accuracy.

In order to achieve the above-mentioned objects, the present invention provides the following means.
The present invention provides an apparatus for detecting brain function comprising an apparatus main body which has a hat shape and which is put on the head of a subject, and a plurality of electrodes for detecting a brain wave signal, the electrodes being mounted in the apparatus main body separated from each other by intervals, and arranged in contact with the scalp, the apparatus main body comprising a plurality of holder pieces in which at least one electrode is mounted, a bendable belt for linking the holder pieces, and a holder piece interval-adjusting apparatus for adjusting a distance between the holder pieces.

According to the present invention, since the apparatus main body comprises a plurality of holder pieces which hold the electrodes and a belt which links the holder pieces, it is possible to configure the apparatus main body such that each holder piece is at a suitable position with respect to the head, by bending the belt to match the shape and size of the head of the subject. By means of arranging the holder pieces at suitable positions with respect to the head, it is possible for the tips of the electrodes which are held in the holder pieces to be brought into contact with the scalp with greater reliability. In addition, according to the present invention, by means of operating the holder piece interval-adjusting apparatus, it is possible to adjust the distance between the holder pieces. Since an electrode is held in each holder piece, it is possible to adjust the distance between electrodes to match the positions determined for the electrodes with respect to the head of a subject, and thereby, it is possible to detect the functional status of the brain with greater accuracy.

According to the above-mentioned invention, a plurality of electrodes may be mounted in at least one holder piece, and an electrode interval-adjusting apparatus for adjusting the intervals between the electrodes in the holder piece may be provided.
Thereby, when the apparatus is mounted on the head of a subject, it is possible to arrange all of the electrodes at predetermined positions.

In addition, in the above-mentioned invention, the holder piece interval-adjusting apparatus may also have a long hole provided in either one of the holder piece and the belt, a through hole provided in the other of the holder piece and the belt, and a fastening apparatus which passes through these holes and fastens the holder piece and the belt together.
By means of fastening the holder piece and the belt together by passing the fastening apparatus through the long hole and the through hole, it is possible to integrally link a plurality of holder pieces. In addition, by means of moving the fastening apparatus within the long hole, it is possible to easily adjust the interval between the holder pieces.

In addition, in the above-mentioned invention, the fastening apparatus may also be provided in the electrode.
By doing this, it is possible for the fastening apparatus, which fixes the holder piece and the belt, to be formed by the electrode, and thereby, the number of parts can be reduced.

In addition, in the above-mentioned invention, a reference position confirmation apparatus for indicating the root of the nose, the occipital node, the preauricular point, etc., may be provided in the above-mentioned holder piece.
When mounted on the head of the subject, setting can be easily carried out using as a reference the root of the nose, the occipital node, the preauricular point, which are indicated by the reference position confirmation apparatus, in order to position each electrode at a predetermined position with respect to the scalp.

In addition, in the above-mentioned invention, calibrations indicating the distance between the electrodes of two holder pieces linked by the belt may be provided in either of the holder pieces or the belt.
When a plurality of electrodes are arranged on the scalp of a subject, it is possible to measure the distance between electrodes by means of the calibrations, and it is possible to set the electrodes more easily and with greater accuracy.

In addition, in the above-mentioned invention, a direction indicator for indicating the direction for mounting on the head may be provided on the holder piece.
By mounting the holder pieces on the head in accordance with the mounting direction indicated by the direction indicator, it is possible to fix the electrodes at appropriate positions with respect to the head of the subject, without mistaking the mounting direction.

By the present invention, it is possible to obtain the effects that by means of being able to adjust the distance between holder pieces in which at least one electrode is held, it is possible to bring the tips of all the electrodes into contact with the scalp with greater certainty, by adaptation to individual differences in the head of a subject, even with respect to different subjects, and that it is possible to detect the functional status of the brain with greater accuracy.

### [Brief Description of Drawings]

[FIG. 1] is a lateral view showing a brain function detection apparatus according to a first embodiment of the present invention.
[FIG. 2] is a lateral view (a), a front view (b), and a planar view (c) of a human head showing the arrangement of electrodes in the brain function detection apparatus of Figure 1.
[FIG. 3] is a longitudinal cross-section showing the fastened condition of the holder piece and the belt in the brain function detection apparatus of FIG. 1.
[FIG. 4] is a partial planar view showing the connected condition for FIG. 3.
[FIG. 5] is a longitudinal cross-section showing a variant of the fastening apparatus of the brain function detection apparatus of FIG. 1.
[FIG. 6] is a front view showing a variant of the brain function detection apparatus of FIG. 1.
[FIG. 7] is a lateral view showing another variant of the brain function detection apparatus of FIG. 1.
[FIG. 8] is a lateral view showing another variant of the brain function detection apparatus of FIG. 1.
[FIG. 9] is a lateral view showing another variant of the brain function detection apparatus of FIG. 1.

### [Best Mode for Carrying Out the Invention]

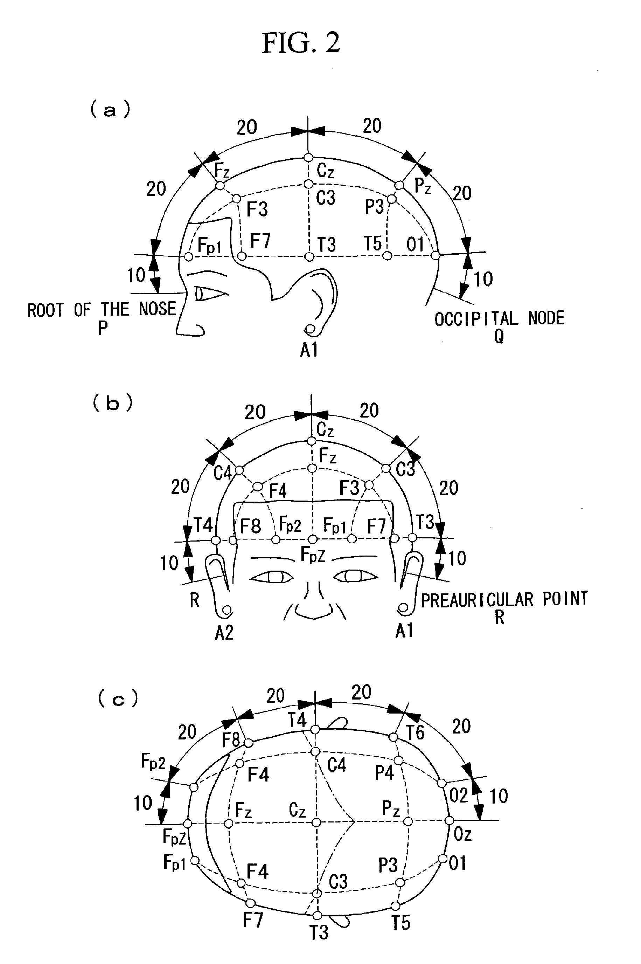

A brain function detection apparatus according to a first embodiment of the present invention will be explained in the following with reference to FIGS. 1 and 2.
As shown in FIG. 1, a brain function detection apparatus 1 according to the present embodiment has a hat-shaped apparatus main body 2 which is mounted on the head B of a subject A being examined and a plurality of electrodes 3 mounted on the apparatus main body 2.
As the electrode 3, as shown in FIGS. 2(a) to (c), normally twenty-one electrodes 3 are arranged on the scalp C at a predetermined ratio using reference positions comprising the root of the nose P, the occipital node Q, and the preauricular point R as a reference.

The apparatus main body 2 has a plurality of holder pieces 4a, 4b, 4c, and a belt 5 which connects these holder pieces 4a, 4b, 4c. The holder pieces 4a, 4b, and 4c are made from resin, or the like, having relative hardness, and the belt 5 is made from a resin, or the like, having relative softness and having relative flexibility.

In the example shown in FIG. 1, as the holder pieces 4a, 4b, and 4c, there are three provided: the front holder piece 4a, the center holder piece 4b, and the rear holder piece 4c. Eight electrodes 3 which are arranged in close contact with the frontal region of the head are mounted in the front holder piece 4a. In addition, five electrodes 3 which are arranged in close contact with the center of the head B in the anteroposterior direction are mounted in the center holder piece 4b. Furthermore, eight electrodes 3 which are arranged in close contact with the rear of the head are mounted in the rear holder piece 4c.

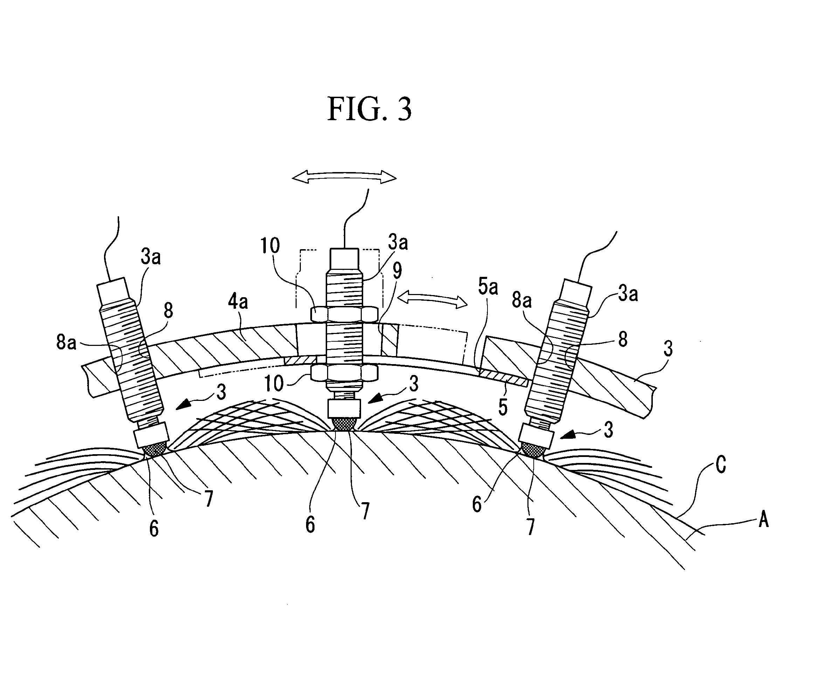

As shown in Figure 3, the electrodes 3 of each of the holder pieces 4a, 4b, and 4c are formed having a long narrow cylindrical shape and have an elastic member 7 containing an electrolyte 6 in the tip which makes contact with the scalp C. By means of the elastic member 7 of the tip making contact with the scalp C and becoming elastically deformed, the electrolyte 6 contained in the elastic member is squeezed out and wets the scalp C, and thereby, it is possible for the electrode plate (not shown in the figure) within the elastic member 7 and the scalp C to become electrically connected.

As shown in Figure 3, the electrode 3 of each holder piece 4a, 4b, and 4c is formed such that it is held in a condition passing through a through hole 8, 9, which passes through the holder piece 4a, 4b, 4c in the thickness direction, and that the elastic member 7 of the tip projects from the inner side of the holder piece 4a, 4b, 4c. Of the eight electrodes 3 of the front holder piece 4a, the two electrodes 3 to the front are formed such that a male screw thread 3a formed on the outer peripheral surface thereof engages a female screw thread 8a formed in the through hole 8 of the front holder piece 4a and thereby it is possible for the two electrodes 3 to be held in such a way that the elastic member 7 of the tip projects to the inner side of the front holder piece 4a. In addition, by varying the fastening position of the male screw thread 3a and the female screw thread 8a, it is possible to change the length of the projection of the tip.

The through holes 9 into which the five electrodes 3 (of the eight electrodes 3 of the front holder piece 4a) to the rear of front holder piece 4a are mounted are formed as long holes which extend in the anteroposterior direction. When a through hole 9 having a long shape which is formed in the front holder piece 4a and a long hole 5a formed in the belt 5 are aligned, an electrode 3 can be inserted through the through hole 9 and the long hole 5a and fixed by the fastening together of the front holder piece 4a and the belt 5 by the nuts 10 which are screwed onto the male screw thread 3a.

In addition, in the same way as for the front two electrodes of front holder piece 4a, the five electrodes 3 of the center holder piece 4b are formed such that they are held in the center holder piece 4b in a condition in which the tips thereof project to the inner side, by means of the fastening of the male screw thread 3a formed in the outer peripheral surface of the electrode 3 and the female screw thread 8a of the through hole 8 formed in the center holder piece 4b. In addition, one end of the belt 5 for fixing the center holder piece 4b to the front holder piece 4a and one end of the belt 5 for fixing center holder piece 4b to the rear holder piece 4c are fixed to the center holder piece 4b.

The through holes 9 into which the five electrodes 3 (of the eight electrodes 3 of the rear holder piece 4c) to the front of the rear holder piece 4c are mounted are also formed as long holes which extend in the anteroposterior direction. When a through hole 9 having a long shape which is formed in the rear holder piece 4c and a long hole 5a formed in the belt 5 are aligned, an electrode 3 can be inserted through the through hole 9 and the long hole 5a and fixed by the fastening together of the rear holder piece 4c and the belt 5 by nuts 10 which are screwed onto the male screw thread 3a.

Reference position-indicating members 11 which indicate each reference point are provided in each of the holder pieces 4a, 4b, and 4c. In the front holder piece 4a, a reference position-indicating member 11 which indicates the root of the nose P is provided. In the center holder piece 4b, a reference position-indicating member 11 which indicates the preauricular point R is provided. In the rear holder piece 4c, a reference position-indicating member 11 which indicates the occipital node Q is provided. These reference position-indicating members 11 are formed such that they extend in directions along the scalp C from each of the holder pieces 4a, 4b, and 4c, and the ends are bent toward the scalp C so as to indicate the respective reference positions.

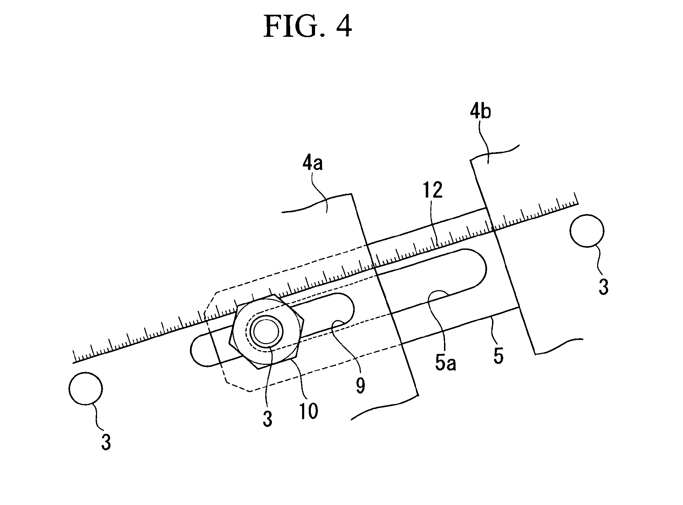

A long hole 5a formed in the longitudinal direction is provided in belt 5. In addition, as shown in FIG. 4, between the electrodes 3 of the holder pieces 4a, 4b, and 4c and in the belt 5, calibrations 12 are provided in a direction which is the anteroposterior direction when mounted on the head B.
In addition, an arrow (direction indicator) 15 showing the direction for mounting the apparatus main body 2 on the head B of the subject A is provided in the front holder piece 4a.

To detect the functional status of the brain of a subject A using the brain function detection apparatus 1 according to the present embodiment constituted in the above-mentioned way, the front holder piece 4a, the center holder piece 4b, and the rear holder piece 4c are arranged on the head B of the subject A, and the distance between the holder pieces 4a, 4b, and 4c are adjusted by means of the belts 5 between the holder pieces 4a, 4b, and 4c. As a result, it is possible to configure the apparatus main body 2, in which the holder pieces 4a, 4b, and 4c are linked, in shapes and sizes which match the heads B of a plurality of subjects A, accommodating individual differences in each subject A.

At this time, when the nuts 10 which fix the electrodes 3 in the holder pieces 4a and 4c are loose, the electrodes can be moved in the longitudinal direction within the through holes 9 and the long holes 5a, and thereby, it is possible to arrange the electrodes such that they match the head B of each subject A. In addition, at this time, with reference to the calibrations 12 and the reference position-indicating members 11 provided in the holder pieces 4a, 4b, and 4c and the belts 5, it is possible to easily set the distance between the electrodes 3.

In addition, when the nuts are being tightened, the amount the tips of the electrodes project from the inner surface of the holder pieces 4a, 4b, and 4c can be adjusted, and the nuts tightened. By means of doing this, it is possible to fix the holder pieces 4a, 4b, and 4c in such a way that the tips of all of the electrodes 3 make contact with the scalp C at the same time.
According to the brain function detection apparatus 1 of the present embodiment, it is possible to arrange in contact with the scalp C the tips of the electrodes 3 in appropriate positions, for a plurality of subjects A, whose heads B differ in shape and size. Consequently, there is the effect that by means of the electrodes 3 which are in contact with the scalp C in a suitable contact condition at suitable positions with respect to the reference positions, it is possible to detect brain wave signals with greater accuracy.

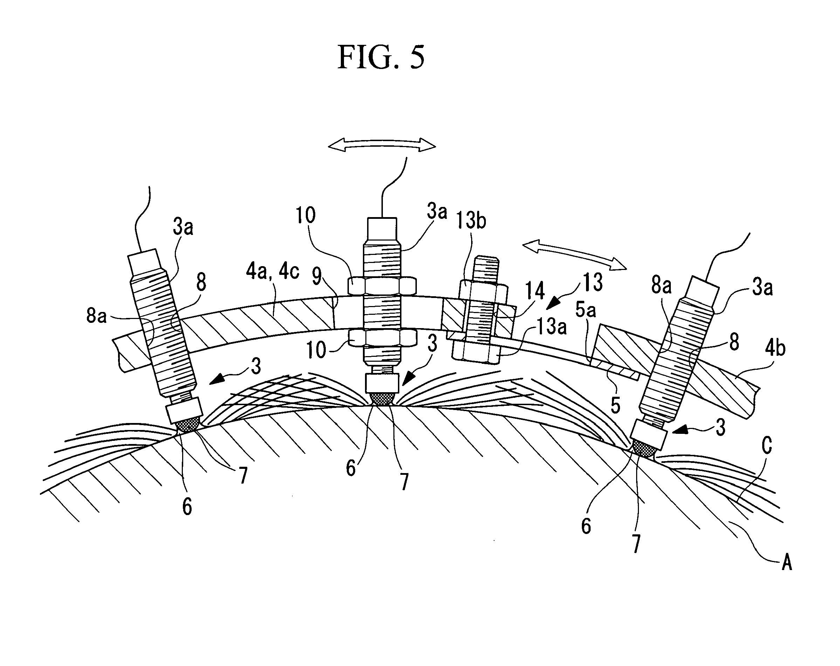

In the brain function detection apparatus 1 according to the present embodiment, the holder pieces 4a and 4c and the belt 5 are fastened together by means of nuts 10 which are screwed onto the male screw thread 3a provided on the electrode 3, but in stead, as shown in FIG.5, the holder pieces 4a to 4c and the belt 5 may be held by means of a fastening apparatus 13 other than the electrodes 3. The fastening device 13, for example, has a bolt 13a which passes through a through hole 14 formed in the holder pieces 4a and 4c, and a nut 13b which is fastened to the bolt 13a, such that the holder pieces 4a and 4c and the belt 5 are fastened together.
Thereby, it is possible to adjust the intervals between the holder pieces 4a, 4b, and 4c by means of the fastening apparatus 13, and to adjust the intervals between the electrodes 3 by means of the male screw thread 3a of the electrode 3 and the nuts 10.

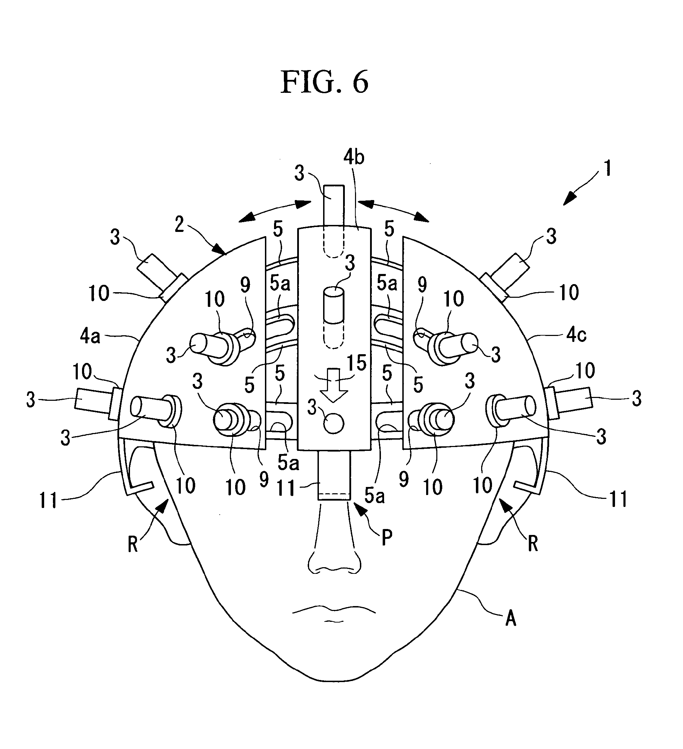

In addition, the brain function detection apparatus 1 according to the present embodiment is exemplified using an apparatus main body 2 which is divided into three holder pieces 4a, 4b, and 4c in the anteroposterior direction of the subject A, but instead, as shown in FIG. 6, the apparatus main body 2 may be divided into three holder pieces 4a, 4b, and 4c in the left-to-right direction of the subject A. In addition, the number of divisions is not limited to three, and the use of any number of divisions of two or more is possible.

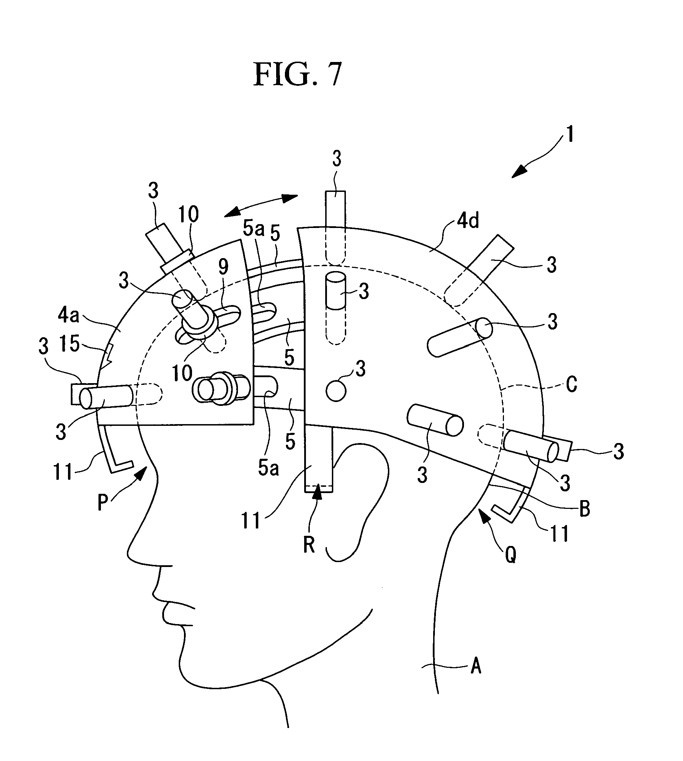

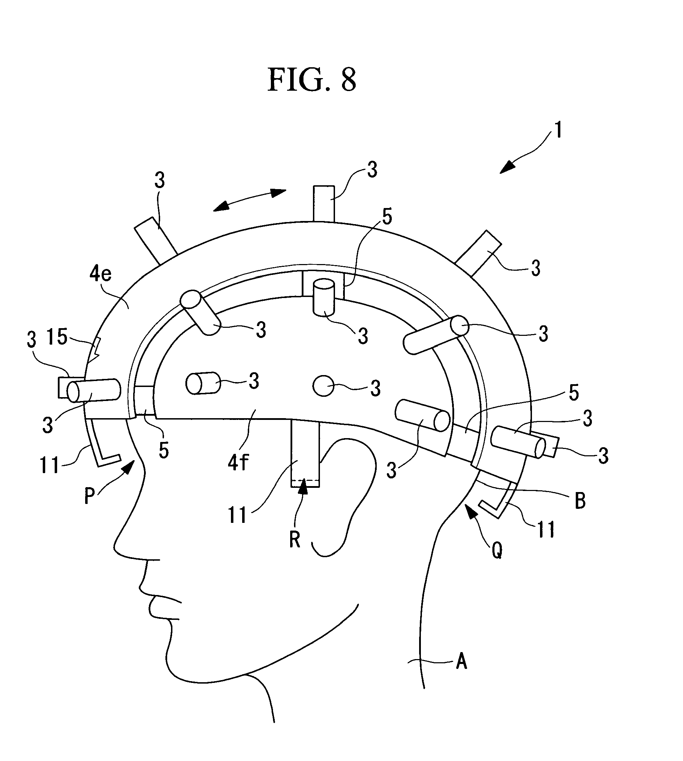

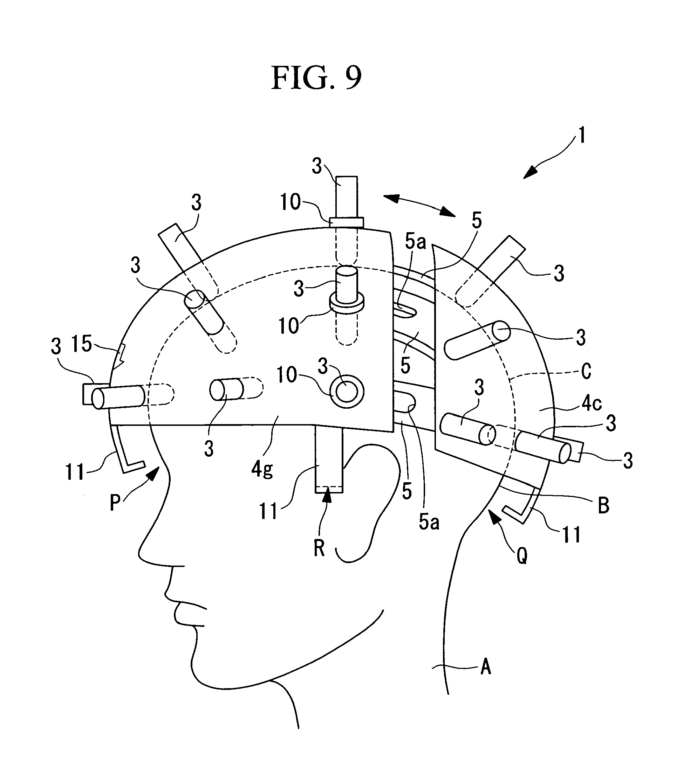

In addition, as shown in FIGS. 7 to 9, the apparatus main body 2 may be divided into holder pieces 4a, 4c, 4d, 4e, 4f, and 4g such that the electrodes are separated for each function of the brain.
For example, the frontal region of the head is the region related to high-level functions such as imagination, prediction, and judgment, motor function, and speech; the temporal region is the region related to auditory function, language recognition, musical cognition, and memory; and the occipital region of the head is the region related to the visual function. Consequently, it is possible to provide holder pieces 4a, 4c, 4d, 4e, 4f, and 4g, such that the frontal region alone may be separated as shown in FIG. 7, the temporal region alone may be separated as shown in FIG. 8, and the occipital region alone may be separated as shown in FIG. 9.

## Claims

1. A brain function detection apparatus, comprising:
an apparatus main body which has a hat shape and which is put on the head of a subject; and
a plurality of electrodes for detecting a brain wave signal, the electrodes being mounted in the apparatus main body separated from each other by intervals and arranged in contact with the scalp;
the apparatus main body having a plurality of holder pieces in which at least one electrode is mounted, a bendable belt for linking the holder pieces, and a holder piece interval-adjusting apparatus for adjusting a distance between the holder pieces.

2. A brain function detection apparatus according to claim 1, comprising a plurality of electrodes mounted in at least one holder piece and an electrode interval-adjusting apparatus for adjusting an interval between electrodes in the holder piece.

3. A brain function detection apparatus according to claim 1, wherein the holder piece interval-adjusting apparatus comprises a long hole provided in either one of the holder piece and the belt, a through hole provided in the other of the holder piece and the belt, and a fastening apparatus which passes through the long hole and the through hole and fastens the holder piece and the belt together.

4. A brain function detection apparatus according to claim 3, wherein the fastening apparatus is provided on the electrode.

5. A brain function detection apparatus according to claim 1, comprising a reference position confirmation apparatus for indicating the root of the nose, the occipital node, the preauricular point, or the like, in the holder piece.

6. A brain function detection apparatus according to claim 1, wherein calibrations indicating a distance between the electrodes of two holder pieces linked by the belt are provided in either one of the holder piece and the belt.

7. A brain function detection apparatus according to claim 1, wherein a direction indicator for indicating a direction for mounting on the head is provided on the holder piece.
